# EUROPEAN PATENT SPECIFICATION

(11) **EP 3 775 139 B1**
(45) Date of publication and mention of the grant of the patent: **24.11.2021**
(21) Application number: 19749187.1
(22) Date of filing: 25.06.2019
(51) Int. Cl.: C11D 3/48, C11D 11/00, G01N 33/36

(54) **EVALUATION METHOD FOR LAUNDRY**
VERFAHREN ZUR BEURTEILUNG VON WÄSCHE
PROCÉDÉ D'ÉVALUATION POUR DU LINGE

(30) Priority: 27.06.2018 EP 18180078
(43) Date of publication of application: 17.02.2021
(73) Proprietor: Unilever IP Holdings B.V., 3013 AL Rotterdam (NL); Unilever Global IP Limited, Wirral, CH62 AZD (GB)
(72) Inventor: MCKEE, Anthony, Wirral Merseyside CH63 3JW (GB)
(74) Representative: Corsten, Michael Allan
(86) International application number: PCT/EP2019/066885
(87) International publication number: WO 2020/002360

(56) References cited:
- US-A1- 2002 178 509
- US-A1- 2009 038 083
- US-B1- 6 379 658

## Description

### Field of the Invention

The invention relates to methods for tracking and evaluation of microbial populations and malodour on laundry articles, and to the preparation of samples for use in such methods.

### Background and Prior Art

Modern laundry detergent compositions have become very effective at removing dirt and stains from laundered articles. However, laundry malodour remains a source of consumer concern, particularly in countries where people wash laundry at low temperatures and/or hang it indoors to dry.

Malodour is defined as an olfactory stimulant which when detected is considered as offensive to the individual. Malodor in a domestic laundry context is thought to be influenced by several factors. These factors include the drying environment, microbial colonisation (biofilms) in washing machines, microbial populations on fabrics and products of microbial metabolism, as well as odorants which attach to fabrics through direct contact with the human body.

US 6,379,658 B1, US 2002/0178509 A1 and US 2009/0038083 A1 disclose a process for reducing or neutralizing malodour from laundry articles.

It would be desirable to develop an improved understanding of at least some of the above factors and how they might interact in practice. In this way, better-targeted and more effective interventions against laundry malodour could be designed.

Whilst successful dirt and stain removal can be assessed visually, microbes and malodours are invisible to the naked eye. Previous evaluation methods used in this context have involved the inoculation of test fabric with high levels of specific microbial strains; or have used actual consumer articles. These methods have problems associated with their implementation, especially on a large scale. For example, isolated and cultured microbial strains require very careful handling techniques to prevent cross contamination during testing. Specific containment protocols may also apply to ensure maximum safety, even though the microbes in question may pose no significant risk to health at their usual levels in their regular environment. As for actual consumer articles, these are difficult to source in large quantities and vary greatly in their level of contamination.

It is an object of the present invention to provide a method for tracking and evaluation of microbial populations and malodour on laundry articles which is reliable and convenient to carry out.

### Summary of the Invention

The present invention provides a method for tracking and evaluation of microbial populations and malodour on laundry articles, comprising the stages of
(i) providing at least one clean, dry sample of pick-up fabric;
(ii) laundering the sample together with soiled laundry articles in a laundry vessel for a laundering period of from 15 minutes to 4 hours;
(iii) after completion of the laundering period, retaining the damp sample in the laundry vessel without further treatment for a retention period of at least 24 hours;
(iv) after completion of the retention period, removing the damp sample from the laundry vessel to dry, and
(v) storing the dried sample without further treatment for a storage period of at least 24 hours;

in which sensory and/or chemical and/or microbiological assessments are carried out on the sample at two or more of the stages (i), (iv), and (v) as defined above.

### Detailed Description and Preferred Embodiments

Stage (i) of the method of the invention requires the provision of at least one clean, dry sample of pick-up fabric.

The term "sample" in the context of this invention denotes a piece designed to represent a larger whole. A small sample, usually taken from existing fabric, is often called a swatch. Swatches of a size from 10 to 20 cm² are convenient for use in the method of the invention.

The pick-up fabric is generally of a type which is representative of the most common fabrics which are encountered in a domestic laundry setting. Such fabrics may be woven, nonwoven or knitted or a blend thereof; and can be made up of natural, synthetic or semi-synthetic fibres or a blend thereof. Cotton, wool, silk and flax are examples of natural fibres. Synthetic and semi-synthetic fibres may be manufactured from plastic granulates or pulp. The most common ones are polyester, polyamide (often called nylon), acrylic and modacrylic, polypropylene, segmented polyurethanes (which are elastic fibres known as elastane) and regenerated cellulose fibres such as viscose, lyocell, modal, acetate and triacetate.

Preferred pick-up fabrics are made up of fibres selected from cotton, polyester, elastane and blends thereof (such as polycotton and polyester elastane); and may be in woven and/or knitted form. Before use, the pick-up fabric may be cleaned if necessary (for example to remove sizing or other substances which may remain on the fabric surface as the result of its manufacture); and dried to remove water remaining on the fabric as a consequence of the cleaning process.

For ease of handling, swatches are preferably attached to a carrier such as a ballast sheet. The term "ballast sheet" in the context of this invention denotes a clean, dry sheet of ballast fabric. Ballast fabrics (also known as "makeweights" or "loading fabrics") are used to make up the wash load to the desired weight for simulating domestic laundering. Suitable ballast fabrics are described in ISO standards (e.g. ISO 6330). Such fabrics include Type I, 100% Cotton ballast, Type II, 50% Cotton/50% Polyester ballast and Type III, 100% Polyester ballast and mixtures thereof.

In a typical method of the invention, a plurality of swatches of pick-up fabric are prepared. Preferably, the plurality of swatches is made up of a range of different types of pick-up fabric. More preferably, the plurality of swatches is made up of a range of different types of pick-up fabric selected from the preferred pick-up fabrics described above. A plurality of ballast sheets is also prepared, and the swatches attached to the ballast sheets. Preferably the swatches are arranged on the ballast sheets in a regular array (such as a grid pattern) for ease of analysis and/or grouped according to fabric type. The number of ballast sheets and the number of swatches attached to each ballast sheet are usually chosen so that the total dry fabric weight per wash load is reasonably typical of a family wash (generally about 1 to about 5kg, such as from about 2 to about 3kg total dry fabric weight per wash load).

In stage (ii) of the method of the invention, the at least one clean, dry sample of pick-up fabric is laundered together with soiled laundry articles in a laundry vessel for a laundering period of from 10 minutes to 4 hours.

The soiled laundry articles will typically include clothing, linens or other household textiles which have been exposed to soiling through normal wear or domestic use. The term "linen" is often used to describe certain types of laundry items including bed sheets, pillow cases, towels, tablecloths, table napkins and uniforms.

Generally, laundering is performed by soaking and/or agitating the laundry in an aqueous wash liquor in the laundry vessel, followed by rinsing the laundry in water.

Usually stage (ii) is performed for a laundering period in the range of from 10 minutes to 2 hours, preferably from 10 minutes to 1 hour and most preferably from 15 to 45 minutes.

Preferably in stage (ii) of the method of the invention, the laundry vessel is an automatic washing machine.

An automatic washing machine for use in the method of the invention may be any conventional automatic washing machine useful for the laundering of fabrics. Such automatic washing machines are those typically found in the home or in businesses such as self-service launderettes where individual consumers can launder their own loads of fabrics.

Washing machines utilize a mechanical agitator to produce agitation of washing and rinsing solutions. Automatic washing machines are also fitted with means for controlling wash and rinse water temperatures and wash and rinse duration. Automatic washing machines can also provide the means for both forming and removing washing and rinsing solutions by using filling and draining apparatus and filling, draining and/or spin cycles An automatic washing machine for use in the method of the invention will generally comprise an external casing; a wash tub connected in floating manner to the casing by one or more suspension devices; a perforated laundry drum for housing laundry for washing, and which is mounted inside the wash tub to rotate about an axis of rotation; and a drive unit connected by a connecting member to the laundry drum to rotate it, on command, about the axis of rotation. A loading/unloading door ensures access to the drum. For domestic use, the drum capacity will generally range from 2 up to 10 kg, more typically up to 6kg of dry cotton laundry.

Automatic washing machines are usually classified into two major groups in terms of the structures and mechanisms; top-loading and front-loading. Top-loading machines have an upright or vertical drum into which fabrics to be laundered are placed. Fabrics are added into the drum, which is usually cylindrical, from the lidded top of the machine. Top-loading machines frequently have a moving agitator element placed along the axis of the drum. Rotation and vertical motion of the agitator serves to intensify the laundering action. Front-loading machines have a drum, also generally cylindrical, which is positioned with the drum axis sideways or in a horizontal position. Fabrics are added into the drum through a door on the front wall of the machine. Front-loading machines typically do not have an agitator element. Mechanical energy is instead imparted to the laundry by the tumbling action formed by the repeated lifting and dropping of the laundry which is implemented by the rotating drum. The drum may also incorporate lifting vanes or similar protrusions to lift the laundry. For domestic use, front-loading automatic washing machines are often preferred due to their superior water and energy efficiency.

Preferably in stage (ii) of the method of the invention, the sample and the soiled laundry articles are placed into the drum of an automatic washing machine, and a wash cycle is run.

The term "wash cycle" in the context of this invention denotes a cycle of operation generally employed in automatic washing machines, in which the machine goes through a series of stages during which water is added, contacted with fabrics being laundered and then extracted from the washing machine drum by spinning. A wash cycle will typically consist of at least a wash stage, a rinse stage and a spin stage. The wash stage, the rinse stage and the spin stage may themselves consist of several steps, such as a fill step, a drain step, a pause step, an agitation step, and any combination thereof. A user may select one of several laundering options (programmes) based upon the type of laundry load being placed in the washing machine (e.g. cotton, wool, synthetic, delicate) and/or the temperature at which the washing must be performed (typically between 30 °C and 90 °C). A wash cycle may also involve one or more additional wash and/or spin and/or rinsing stages respectively, depending on the programme selected.

A detergent is normally used in the wash stage to assist in and promote the removal of soil from the fabrics being laundered. In automatic washing machines, the dose of detergent is typically put into a dispenser and from there it is flushed into the machine by the water flowing into the machine, thereby forming the wash liquor. Alternatively, the dose of detergent may be added directly into the drum. Dosages for a typical front-loading washing machine (using 10 to 15 litres of water to form the wash liquor) may range from 10 ml to 60 ml, preferably 15 to 40 ml. Dosages for a typical top-loading washing machine (using from 40 to 60 litres of water to form the wash liquor) may be higher, e.g. up to 100 ml. Lower dosages of detergent (e.g. 50 ml or less) may be used for hand washing methods (using 1 to 10 litres of water to form the wash liquor). Any input of water during the rinse stage(s) is not included when determining the volume of the wash liquor.

Preferably in stage (ii) of the method of the invention, the total quantity of fabric (pick-up fabric plus soiled laundry articles) relative to wash liquor ranges from 8 to 20g fabric per litre of wash liquor and preferably ranges from 10 to 15g fabric per litre of wash liquor.

A detergent for use in the invention may suitably be in liquid or particulate form, or a mixture thereof. The term "particulate" in the context of this invention denotes free-flowing or compacted solid forms such as powders, granules, pellets, flakes, bars, briquettes or tablets. The term "liquid" in the context of this invention denotes that a continuous phase or predominant part of the composition is liquid, and that the composition is flowable at 15°C and above. Accordingly, the term "liquid" may encompass emulsions, suspensions, and compositions having flowable yet stiffer consistency, known as gels or pastes. The viscosity may suitably range from 200 to 10,000 mPa.s at 25°C at a shear rate of 21 sec⁻¹. This shear rate is the shear rate that is usually exerted on the liquid when poured from a bottle.

A detergent for use in the invention will generally comprise one or more surfactants, which may be non-ionic including semi-polar and/or anionic and/or cationic and/or zwitterionic. The surfactants are typically present at a level of from 0.1 to 60% (by weight based on the total weight of the composition).

When included, the detergent will usually contain from about 1 to about 40% (by weight based on the total weight of the composition) of an anionic surfactant such as linear alkylbenzene sulfonate (preferably C₁₁ to C₁₅ linear alkyl benzene sulfonate) and sodium lauryl ether sulfate (preferably C₁₀ to C₁₈ alkyl sulfate ethoxylated with an average of 1 to 3 EO), or mixtures thereof.

When included, the detergent will usually contain from 0.2 to 40% (by weight based on the total weight of the composition) of a non-ionic surfactant such as aliphatic C₈ to C₁₈, more preferably C₁₂ to C₁₅ primary linear alcohol ethoxylates with an average of from 3 to 20, more preferably from 5 to 10 moles of ethylene oxide per mole of alcohol, or mixtures thereof.

The detergent may contain from 0 up to 65% (by weight based on the total weight of the composition) of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates and layered silicates (such as crystalline sodium disilicate), or mixtures thereof.

The detergent may comprise one or more polymers. Examples are carboxymethylcellulose, poly(vinylpyrrolidone), poly(ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers, or mixtures thereof.

The detergent may contain a bleaching system which may comprise a H₂O₂ source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

A liquid detergent may be aqueous, typically containing up to 70% (by weight based on the total weight of the composition) water and from 0 to 30% (by weight based on the total weight of the composition) non-aqueous carriers such as C1 to C5 monohydric alcohols, C2 to C6 diols, C3 to C9 triols, polyethylene glycols having a weight average molecular weight (M_{w}) ranging from 200 to 600, C1 to C3 alkanolamines and alkyl aryl sulfonates having up to 3 carbon atoms in the lower alkyl group, or mixtures thereof.

The detergent may also contain other conventional detergent ingredients such as enzymes and enzyme stabilizing agents, clays, foam boosters, suds suppressors, anticorrosion agents, soil-suspending agents, anti-soil redeposition agents, dyes, bactericides, optical brighteners, hydrotropes, tarnish inhibitors, or perfumes.

Other fabric care additives such as softeners, feel modifiers and/or anti-wrinkle agents may be contacted with the fabrics being laundered during the rinse stage.

A preferred type of wash cycle for stage (ii) of the method of the invention is a delicates programme characterised by a wash temperature ranging from 20 to 40°C, from 1 to 3 rinses and a spin speed of less than 1000 rpm, such as from 500 to 800 rpm. The wash stage duration of a such a programme is usually from about 30 to 40 minutes with the wash cycle overall duration being from 50 to 70 minutes.

In a preferred method according to the invention, after completion of the wash cycle (such as the delicates programme described above), the laundry articles are removed from the drum with the damp sample being retained inside the drum, preferably with the machine door or lid closed.

According to stage (iii) of the method of the invention, the damp sample is retained in the laundry vessel (preferably inside the drum of an automatic washing machine) without further treatment for a retention period of at least 24 hours.

Preferably the retention period is at least 36 hours, such as from 36 to 72 hours and ideally about 48 hours. The sample remains damp during the retention period. The moisture level of the damp sample will depend on the initial moisture level of the fabric after completion of the wash cycle. This may range from about 10 to 20% (based on dry fabric weight) for polyester fabrics up to about 40 to 65% (based on dry fabric weight) for cotton fabrics.

In stage (iv) of the method of the invention, after completion of the retention period the damp sample is removed from the laundry vessel to dry. Generally, the damp sample will be dried to a residual moisture level of no more than about 5% (based on dry fabric weight), preferably from 0 to 2.5% (based on dry fabric weight). Typically, the damp sample is allowed to air dry for a period of about 6 to 18 hours. The drying conditions (such as temperature and relative humidity) may be chosen to reflect, for example, a typical domestic laundry drying environment such as an indoor drying rack. For example, drying temperature may suitably range from 20 to 25°C and drying relative humidity (RH) may suitably range from 40 to 65%.

In stage (v) of the method of the invention, the dried sample is stored without further treatment for a storage period of at least 24 hours, preferably at least 3 days and more preferably from 4 to 8 days. The storage conditions (such as temperature and relative humidity) may be chosen to reflect, for example, a typical domestic laundry storage environment such as an airing cupboard. For example, storage temperature may suitably range from 20 to 30°C, and storage relative humidity (RH) may suitably range from 65 to 85%.

Sensory and/or chemical and/or microbiological assessments are carried out on the sample at two or more of the stages (i), (iv) and (v) as defined above. Preferably, the assessments are carried out at all three stages (i), (iv) and (v).

A range of techniques are available to the skilled worker for the assessment of sensory, chemical and microbiological properties respectively. For example, malodour assessments may be carried out using trained panellists to determine hedonic value, intensity, and qualitative odour characteristics. Malodour causing substances may be quantitatively detected by using methods such as column chromatography. Microbial communities may be measured using plate count techniques involving the growth of colonies on a nutrient agar surface.

After completion of the storage period of stage (v) of the method of the invention, the dried, stored samples may be used in further tests, as desired by the tester. A preferred method according to the invention comprises at least the following further stages:
(vi) after completion of the storage period, laundering the dried, stored sample in a laundry vessel for a laundering period of from 15 minutes to 4 hours;
(vii) after completion of the laundering period, removing the damp sample from the laundry vessel to dry, and
(viii) storing the dried sample without further treatment for a storage period of at least 24 hours.

Preferably in stage (vi) of the preferred method of the invention, the sample is laundered by placing it in the drum of an automatic washing machine together with clean, dry ballast sheets and running a wash cycle. Generally, sufficient clean, dry ballast sheets are added so that the total dry fabric weight per wash load is reasonably typical of a family wash (generally 1 to 5kg, such as from 2 to 3kg total dry fabric weight per wash load). The wash cycle used may be the same programme as that selected for stage (ii), or may be different.

In stage (vii) of the preferred method of the invention, the damp sample is removed from the laundry vessel to dry. The damp sample may be removed straight after completion of the laundering period in stage (vi), or alternatively after a retention period of up to 24 hours (during which the damp sample is retained inside the laundry vessel without further treatment).

In stage (viii) of the preferred method of the invention, the dried sample is stored without further treatment for a storage period of at least 24 hours, preferably at least 3 days and more preferably from 4 to 8 days. The storage conditions (such as temperature and relative humidity) may be the same as those selected for stage (v), or may be different.

Sensory and/or chemical and/or microbiological assessments are preferably carried out on the sample at one or more of the stages (vi), (vii) and (viii) as defined above and are more preferably carried out on the sample at two or more of the stages (vi), (vii) and (viii) as defined above.

The further stages (vi), (vii) and (viii) as defined above may usefully be repeated under varying wash conditions. Examples of conditions which may be varied in this manner include detergent type, fabric type, wash water hardness, and wash cycle variables such as temperature, duration and spin speed. Comparison of the assessment results enables the user to evaluate the impact of varying the selected condition.

Advantages of the method of the invention include increased sample preparation capacity as well as improved sample consistency and reproducibility.

Therefore, the method of the invention may help to provide a more reliable, meaningful and comprehensive evaluation of laundry malodour and the factors which influence it.

The invention is further illustrated by reference to the following Example.

### EXAMPLE

### Preparation phase

The following wash load was prepared for laundering in an automatic washing machine (c.2.5 kg dry fabric weight per machine):
16 soiled bath towel quarters (naturally soiled towels are used as a source of soil and are collected from consumers after 2 weeks use with a single wash and dry in between before collection)
15 polyester/cotton ballast sheets to which swatches (10cm x 10cm) of pick-up fabrics were attached (using a tagger):
   - *25 knitted polyester elastane (PE) pick-up fabrics*
   - *25 knitted polyester (KP) pick-up fabrics*
   - *25 knitted cotton (KC) pick-up fabrics*
   - *25 woven cotton (WC) pick-up fabrics*
   - *25 woven polycotton (WPC) pick-up fabrics*

The load was washed with an unfragranced liquid detergent on a silk cycle (30 mins, 2 rinses, 700 rpm spin, 30°C) using 26FH water.

On completion of the wash cycle, the damp pick-up fabric swatches were kept inside the machine for a retention period of 48 hours.

On completion of the retention period, the damp pick-up fabric swatches were removed from the machine. Microbiological measurements were made on the damp pick-up fabric swatches (bacterial and fungal loading via TVCs & selective media; bacterial identification via sequencing). Volatile or semi-volatile compounds originating from the damp pick-up fabric swatches were analysed by a GC-IMS technique (in which the compounds were selectively separated by the GC column and then introduced into the ionization region of the IMS where they are converted into gas phase ions and detected according to their mobility, ion cross-section and charge).

The damp pick-up fabric swatches were hung up to air dry overnight at 22°C/45% RH, after which microbiological and analytical measurements were made on the dried pick-up fabric swatches. Sensory properties of the dried pick-up fabric swatches were also assessed by trained panellists (malodour score assigned using a scale of 0-5 in addition to odour characteristics).

The dried pick-up fabric swatches were stored for 6 days at 30°C/85% RH, after which microbiological, analytical and sensory measurements were made on the dried, stored pick-up fabric swatches.

### Washing phase

The dried, stored pick-up fabric swatches were then machine washed again together with clean, dry WPC ballast sheets (to form a load of 2.5 kg dry fabric weight per machine):
The load was washed on a cotton (Superquick) cycle (1-hour, 1200 rpm spin, 40°C) using 26FH water.

The detergent used was either a liquid (Lysoform® laundry liquid) or a powder (Persil® Bio powder).

On completion of the wash cycle, the damp pick-up fabric swatches were removed from the machine. Microbiological and analytical measurements were made on the damp pick-up fabric swatches straight after removal from the machine.

The damp pick-up fabric swatches were hung up to air dry overnight at 22°C/45% RH, after which microbiological, analytical and sensory measurements were made on the dried pick-up fabric swatches.

The dried pick-up fabric swatches were stored for 7 days at 30°C/85% RH, after which microbiological, analytical and sensory measurements were made on the dried, stored pick-up fabric swatches.

### Analysis

The dataset obtained was used to chart the variation in log bacteria (TVC) and mean malodour score at the different stages (damp fabric, dried fabric and dried, stored fabric) across the preparation phase and subsequently across the washing phase.

The dataset was also used to evaluate the influence of both swatch fabric type and detergent type on the microbial and malodour profile across the different stages.

The method of the invention may therefore be useful in exploring how various aspects of the domestic laundry process might be tailored to improve antimicrobial or antimalodour performance.

## Claims

1. A method for tracking and evaluation of microbial populations and malodour on laundry articles, comprising the stages of
(i) providing at least one clean, dry sample of pick-up fabric;
(ii) laundering the sample together with soiled laundry articles in a laundry vessel for a laundering period of from 15 minutes to 4 hours;
(iii) on completion of the laundering period, retaining the damp sample in the laundry vessel without further treatment for a retention period of at least 24 hours;
(iv) on completion of the retention period, removing the damp sample from the laundry vessel to dry, and
(v) storing the dried sample without further treatment for a storage period of at least 24 hours;
in which sensory and/or chemical and/or microbiological assessments are carried out on the sample at two or more of the stages (i), (iv) and (v) as defined above.

2. A method according to claim 1, in which the sample of pick-up fabric is in the form of a 10 to 20 cm² sized swatch.

3. A method according to claim 1 or claim 2, in which the pick-up fabric is made up of fibres selected from cotton, polyester, elastane and blends thereof (such as polycotton and polyester elastane); and is in woven and/or knitted form.

4. A method according to any one of claims 1 to 3, in which a plurality of swatches of pick-up fabric are arranged on ballast sheets in a regular array and/or grouped according to fabric type.

5. A method according to any one of claims 1 to 4, in which in stage (ii) the sample and the soiled laundry articles are placed into the drum of an automatic washing machine and a wash cycle is run.

6. A method according to claim 5, in which in stage (iii) the damp sample is retained inside the drum, with the machine door or lid closed, for a retention period of from 36 to 72 hours.

7. A method according to any one of claims 1 to 6, in which in stage (iv) the damp sample is allowed to air dry for a period of 6 to 18 hours at a temperature of from 20 to 25°C, and a relative humidity (RH) of from 40 to 65%.

8. A method according to any one of claims 1 to 7, in which in stage (v) the dried sample is stored for a period of 4 to 8 days at a temperature from 20 to 30°C, and a relative humidity (RH) of from 65 to 85%.

9. A method according to any one of claims 1 to 8, in which sensory and/or chemical and/or microbiological assessments are carried out on the sample at stages (i), (iv) and (v).

10. A method according to any one of claims 1 to 9, which comprises at least the following further stages:
(vi) after completion of the storage period, laundering the dried, stored sample in a laundry vessel for a laundering period of from 15 minutes to 4 hours;
(vii) after completion of the laundering period, removing the damp sample from the laundry vessel to dry, and
(viii) storing the dried sample without further treatment for a storage period of at least 24 hours.

## Patentansprüche

1. Verfahren zur Verfolgung und Bewertung von mikrobiellen Populationen und schlechtem Geruch auf Wäschestücken, umfassend die folgenden Stufen
(i) Bereitstellen von mindestens einer sauberen, trockenen Probe von Aufnahmestoff;
(ii) Waschen der Probe zusammen mit verschmutzten Wäschestücken in einem Wäschebehältnis für eine Waschdauer von 15 Minuten bis 4 Stunden;
(iii) Aufbewahren der feuchten Probe in dem Wäschebehältnis nach Ablauf der Waschdauer ohne weitere Behandlung für eine Aufbewahrungsdauer von mindestens 24 Stunden;
(iv) Entnahme der feuchten Probe aus dem Wäschebehältnis nach Ablauf der Aufbewahrungsdauer zum Trocknen und
(v) Lagern der getrockneten Probe ohne weitere Behandlung für eine Lagerdauer von mindestens 24 Stunden;
wobei sensorische und/oder chemische und/oder mikrobiologische Bewertungen an der Probe in zwei oder mehr der Stufen (i), (iv) und (v) wie vorstehend definiert durchgeführt werden.

2. Verfahren nach Anspruch 1, bei dem die Probe des Aufnahmestoffs in Form eines Stoffmusters mit einer Größe von 10 bis 20 cm² vorliegt.

3. Verfahren nach Anspruch 1 oder Anspruch 2, bei dem der Aufnahmestoff aus Fasern gebildet ist, die ausgewählt sind aus Baumwolle, Polyester, Elastan und Mischungen davon (wie z.B. Polycotton und Polyester-Elastan); und in gewebter und/oder gestrickter Form vorliegt.

4. Verfahren nach irgendeinem der Ansprüche 1 bis 3, bei dem eine Mehrzahl von Stoffmustern von Aufnahmestoff auf Ballastflächenkörpern in einer regelmäßigen Anordnung angeordnet und/oder nach Stofftyp gruppiert sind.

5. Verfahren nach irgendeinem der Ansprüche 1 bis 4, bei dem in Stufe (ii) die Probe und die verschmutzten Wäschestücke in die Trommel einer automatischen Waschmaschine gegeben werden und ein Waschzyklus durchgeführt wird.

6. Verfahren nach Anspruch 5, bei dem in Stufe (iii) die feuchte Probe in der Trommel für eine Aufbewahrungsdauer von 36 bis 72 Stunden aufbewahrt wird, wobei die Tür oder der Deckel der Maschine geschlossen ist.

7. Verfahren nach irgendeinem der Ansprüche 1 bis 6, bei dem man in Stufe (iv) die feuchte Probe für einen Zeitraum von 6 bis 18 Stunden bei einer Temperatur von 20 bis 25 °C und einer relativen Feuchtigkeit (rF) von 40 bis 65% lufttrocknen lässt.

8. Verfahren nach irgendeinem der Ansprüche 1 bis 7, bei dem in Stufe (v) die getrocknete Probe für einen Zeitraum von 4 bis 8 Tagen bei einer Temperatur von 20 bis 30 °C und einer relativen Feuchtigkeit (rF) von 65 bis 85% gelagert wird.

9. Verfahren nach irgendeinem der Ansprüche 1 bis 8, bei dem sensorische und/oder chemische und/oder mikrobiologische Bewertungen an der Probe in den Stufen (i), (iv) und (v) durchgeführt werden.

10. Verfahren nach irgendeinem der Ansprüche 1 bis 9, das mindestens die folgenden weiteren Stufen umfasst:
(vi) Waschen der getrockneten, gelagerten Probe nach Ablauf der Lagerdauer in einem Wäschebehältnis für eine Waschdauer von 15 Minuten bis 4 Stunden;
(vii) Entnahme der feuchten Probe aus dem Wäschebehältnis nach Ablauf der Waschdauer zum Trocknen, und
(viii) Lagern der getrockneten Probe ohne weitere Behandlung für eine Lagerdauer von mindestens 24 Stunden.

## Revendications

1. Procédé pour le suivi et l'évaluation de populations microbiennes et mauvaise odeur sur des articles de blanchisserie, comprenant les étapes de :
(i) fourniture d'au moins un échantillon propre, sec de textile recueilli ;
(ii) blanchissage de l'échantillon avec des articles de blanchisserie sales dans une cuve de blanchisserie sur une période de blanchissage de 15 minutes à 4 heures ;
(iii) à l'achèvement de la période de blanchissage, la retenue de l'échantillon humide dans la cuve de blanchisserie sans autre traitement sur une période de rétention d'au moins 24 heures ;
(iv) à l'achèvement de la période de rétention, prélèvement de l'échantillon humide de la cuve de blanchisserie pour séchage, et
(v) stockage de l'échantillon séché sans autre traitement sur une période de stockage d'au moins 24 heures ;
dans lequel les analyses sensorielles et/ou chimiques et/ou microbiologiques sont réalisées sur l'échantillon dans deux des étapes (i), (iv) et (v) ou plus comme définies ci-dessus.

2. Procédé selon la revendication 1, dans lequel l'échantillon de textile recueilli est dans la forme d'un échantillon de dimension de 10 à 20 cm².

3. Procédé selon la revendication 1 ou revendication 2, dans lequel le textile recueilli est constitué de fibres choisies parmi du coton, polyester, élasthanne, et combinaisons de ceux-ci, (tels que polycoton et polyester élasthanne) ; et est dans une forme tissée et/ou tricotée.

4. Procédé selon l'une quelconque des revendications 1 à 3, dans lequel plusieurs échantillons de textile recueilli sont disposés sur des feuilles de lest dans une rangée régulière et/ou groupés selon le type de textile.

5. Procédé selon l'une quelconque des revendications 1 à 4, dans lequel dans l'étape (ii) l'échantillon et les articles de blanchisserie sales sont placés dans le tambour d'une machine à laver automatique et un cycle de lavage est lancé.

6. Procédé selon la revendication 5, dans lequel dans l'étape (iii) l'échantillon humide est retenu à l'intérieur du tambour, avec la porte ou le couvercle de machine fermée, sur une période de rétention de 36 à 72 heures.

7. Procédé selon l'une quelconque des revendications 1 à 6, dans lequel dans l'étape (iv) l'échantillon humide est laissé à sécher à l'air sur une période de 6 à 18 heures à une température de 20 à 25°C, et une humidité relative (RH) de 40 à 65 %.

8. Procédé selon l'une quelconque des revendications 1 à 7, dans lequel dans l'étape (v) l'échantillon séché est stocké sur une période de 4 à 8 jours à une température de 20 à 30°C, et une humidité relative (RH) de 65 à 85 %.

9. Procédé selon l'une quelconque des revendications 1 à 8, dans lequel des analyses sensorielles et/ou chimiques et/ou microbiologiques sont réalisées sur l'échantillon dans les étapes (i), (iv) et (v).

10. Procédé selon l'une quelconque des revendications 1 à 9, qui comprend au moins les autres étapes suivantes :
(vi) à l'achèvement de la période de stockage, blanchissage de l'échantillon séché, stocké dans une cuve de blanchisserie sur une période de blanchissage de 15 minutes à 4 heures ;
(vii) à l'achèvement de la période de blanchissage, prélèvement de l'échantillon humide de la cuve de blanchisserie pour séchage, et
(viii) stockage de l'échantillon séché sans autre traitement sur une période de stockage d'au moins 24 heures.
